# EUROPEAN PATENT APPLICATION

(11) **EP 1 612 206 A1**
(43) Date of publication of application: **04.01.2006**
(21) Application number: 04723845.6
(22) Date of filing: 26.03.2004
(51) Int. Cl.: C07D 207/16, A61K 31/401, A61P 3/04, A23L 1/30, A23K 1/16

(54) **ANTI-OBESITY AGENT**

(30) Priority: 28.03.2003 JP 2003089985
(71) Applicant: KYOWA HAKKO KOGYO CO., LTD., Chiyoda-ku, Tokyo 100-8185 (JP)
(72) Inventor: KAMIYA, Toshikazu, c/o Healthcare Research Lab., Tsukuba-shi, Ibaraki 305-0841 (JP); SHIRAI, Akio, Head Office, 1-chome, Chiyoda-ku, Tokyo 100-8185 (JP)
(74) Representative: Vossius & Partner
(86) International application number: PCT/JP2004/004310
(87) International publication number: WO 2004/087657

(57) **Abstract**

An object of the present invention is to provide an anti-obesity agent, or to provide foods and drinks, food and drink additives, and feeds or feed additives for anti-obesity.

In order to achieve such an object, the present invention provides an anti-obesity agent comprising hydroxyproline or an N-acyl derivative of hydroxyproline or a pharmaceutically acceptable salt thereof or foods and drinks, food and drink additives, feeds or feed additives for anti-obesity comprising the same.

## Description

### Technical Field

The present invention relates to an anti-obesity agent and also to foods and drinks, food and drink additives, feeds and feed additives for anti-obesity.

### Background Art

As anti-obesity agents, those which suppress feeding center such as mazindol, those which suppress appetite and enhance feeling of fullness centrally such as sibutramine, those which suppress absorption of fat in stomach and small intestine such as orlistat have been known. However, since drugs which are able to be used for a long period are few, it has been desired that choices for anti-obesity agents increase as the demand for anti-obesity agent will increase in future.

Hydroxyproline widely occurs in nature as a major amino acid component of collagen and its N-acetyl derivative is used as an anti-inflammatory agent. It has also been used as a material for synthesis of various medicaments such as antibiotic substances of carbapenem type, blood pressure depressant, anti-asthma agent, improving agent for peripheral circulation and blood coagulation inhibitor. Further, due to its functional characteristic of having moisturizing property, it has been used for cosmetics as well (*Bioscience and Industry,* 1998, volume 56, no. 1, pages 11 to 16). It has also been used as a food additive for adjustment of quality of taste and improvement in taste of fruit juice, refreshing soft drink and commonly used food or as a material for flavor ("Commentary for Official Formulary of Food Additives", Seventh Edition, published by Hirokawa Shoten, 1998, pages D-1114 to 1115).

With regard to a pharmacological action of hydroxyproline, an action for suppressing aging of the skin and an action for improving skin quality (WO 00/51561; Japanese Published Unexamined Patent Application No. 080321/2002), anti-inflammatory action, anti-rheumatic action, analgesic action and wound-healing action (Japanese Published Unexamined Patent Application No. 337526/1996) have been known, and there has been no report for an action for anti-obesity.

### Disclosure of the Invention

An object of the present invention is to provide an anti-obesity agent and also to provide foods and drinks, food and drink additives, feeds and feed additives for anti-obesity.

The present invention relates to the following (1) to (7).
(1) An anti-obesity agent, which comprises as an active ingredient, hydroxyproline or N-acyl derivative of hydroxyproline or a pharmaceutically acceptable salt thereof.
(2) A food and drink or a food and drink additive for anti-obesity, which comprises as an active ingredient, hydroxyproline or N-acyl derivative of hydroxyproline or a pharmaceutically acceptable salt thereof.
(3) A feed or a feed additive for anti-obesity, which comprises as an active ingredient, hydroxyproline or N-acyl derivative of hydroxyproline or a pharmaceutically acceptable salt thereof.
(4) A method for suppressing obesity, which comprises administering hydroxyproline or N-acyl derivative of hydroxyproline or a pharmaceutically acceptable salt thereof.
(5) Use of hydroxyproline or N-acyl derivative of hydroxyproline or a pharmaceutically acceptable salt thereof for the manufacture of an anti-obesity agent.
(6) Use of hydroxyproline or N-acyl derivative of hydroxyproline or a pharmaceutically acceptable salt thereof for the manufacture of foods and drinks or food and drink additives for anti-obesity.
(7) Use of hydroxyproline or N-acyl derivative of hydroxyproline or a pharmaceutically acceptable salt thereof for the manufacture of feeds or feed additives for anti-obesity.

Hydroxyproline widely occurs in nature as a major amino acid component of collagen and as an amino acid component of elastin. It has been known that there exist eight kinds of stereoisomers of natural hydroxyproline which are distinct from one another, depending on whether proline is the D-form or the L-form, whether the hydroxyl group is at the 3-position or the 4-position, and whether the stereoisomer is the cis-form or the trans-form. Specific examples thereof are mentioned as cis-4-hydroxy-L-proline, cis-4-hydroxy-D-proline, cis-3-hydroxy-L-proline, cis-3-hydroxy-D-proline, trans-4-hydroxy-L-proline, trans-4-hydroxy-D-proline, trans-3-hydroxy-L-proline and trans-3-hydroxy-D-proline.

Although hydroxyproline of any such structure is able to be used in the present invention, trans-4-hydroxy-L-proline is preferably used.

Hydroxyproline is able to be produced by subjecting collagen derived from animals such as pig and cow to acid hydrolysis and purifying the hydrolysate according to a conventional method. However, hydroxyproline produced using microorganisms is preferably used.

Useful microorganisms include those belonging to the genus selected from the group consisting of the genus *Amycolatopsis,* the genus *Dactylosporangium* and the genus *Streptomyces* or those into which a proline 3-hydroxylase gene or a proline 4-hydroxylase gene derived from these microorganisms has been introduced. Introduction of a proline 3-hydroxylase gene or a proline 4-hydroxylase gene derived from a microorganism belonging to the genus selected from the group consisting of the genus *Amycolatopsis,* the genus *Dactylosporangium* and the genus *Streptomyces* into a microorganism can be carried out according to the methods described in Molecular Cloning, A Laboratory Manual, Second Edition, Cold Spring Harbor Laboratory Press (1989), Current Protocols in Molecular Biology, John Wiley & Sons (1987-1997), etc.

Furthermore, trans-4-hydroxy-L-proline is able to be produced using proline 4-hydroxylase isolated from a microorganism belonging to the genus *Amycolatopsis* or the genus *Dactylosporangium* (Japanese Published Unexamined Patent Application No. 313179/1995), and cis-3-hydroxy-L-proline is able to be produced using proline 3-hydroxylase isolated from a microorganism belonging to the genus *Streptomyces* (Japanese Published Unexamined Patent Application No. 322885/1995) [*Bioindustry,* 14, 31 (1997)].

N-acyl derivative of hydroxyproline to be used in the present invention includes those N-acyl derivatives of various hydroxyproline stereoisomers as described above. The acyl group of said N-acyl derivative is not particularly limited, however, preferred is acyl group having 2 - 23 carbon atoms, more preferred is acyl group having 2 - 12 carbon atoms and particularly preferred is acyl group having 2 - 6 carbon atoms. Examples of the acyl group are acetyl, propionyl, butyryl, isobutyryl, valeryl, hexanoyl, heptanoyl, octanoyl, eicosanoyl, tricosanoyl, and the like.

N-acyl derivative of hydroxyproline is able to be produced from hydroxyproline by a known method mentioned, for example, in WO 00/51561, etc.

The resulting N-acyl derivative of hydroxyproline is able to be purified by a common purifying method such as crystallization and chromatography.

As the pharmaceutically acceptable salt of hydroxyproline or N-acyl derivative of hydroxyproline, mention may be made of alkali metal salts such as sodium salts, potassium salts, etc., alkaline earth metal salts such as magnesium salts, calcium salts, etc., ammonium salts such as ammonium, tetramethylammonium, etc., organic amine addition salts to which morpholine, piperidine, etc. and the like.

The anti-obesity agent of the present invention is a pharmaceutical preparation comprising, as an active ingredient, hydroxyproline or N-acyl derivative of hydroxyproline or a salt thereof either solely or in a mixed state or as a mixture with other ingredients for any other treatment.

Such a pharmaceutical preparation is able to be prepared by mixing the active ingredient with one or more pharmaceutically acceptable carriers followed by subjecting to any method which has been well known in the technical field of pharmaceutical preparations.

In administering the preparation, it is desirable to select a route of administration that is the most effective in the treatment and its examples are oral administration and parenteral administrations such as intravenous, intraperitoneal or subcutaneous administration, and an oral administration is preferred.

With regard to the dosage form, any of oral preparation such as tablets, diluted powder, granules, pill, suspensions, emulsion, infusion/decoction, capsules, syrup, liquid, elixir, extract, tincture, fluid extract, etc. and parenteral preparation such as injection, drip infusion, cream, suppository, etc. may be used and an oral preparation is preferably used.

In the manufacture of an oral preparation, it is possible to use additives such as excipient, binder, disintegrating agent, lubricant, dispersing agent, suspending agent, emulsifier, diluting agent, buffer, antioxidant and cell suppressor.

A liquid preparation such syrup which is appropriate for oral administration is able to be prepared by addition of water, saccharide such as sucrose, sorbitol, fructose, etc., glycol such as polyethylene glycol, propylene glycol, etc., oil such as sesame oil, olive oil, soybean oil, etc., antiseptic such as p-hydroxybenzoate, etc., preservative such as p-hydroxybenzoate derivatives (e.g., methyl p-hydroxybenzoate), sodium benzoate, etc., flavor such as strawberry flavor, peppermint, etc. and the like.

Tablets, powder, granule, etc. which are suitable for oral administration are able to be prepared by addition of saccharide such as lactose, sugar, glucose, sucrose, mannitol, sorbitol, etc., starch such as potato, wheat, corn, etc., inorganic substance such as calcium carbonate, calcium sulfate, sodium hydrogen carbonate, sodium chloride, etc., excipient such as crystalline cellulose, plant powder (e.g., powdered licorice and powdered gentian), etc., disintegrating agent such as starch, agar, powdered gelatin, crystalline cellulose, carmellose sodium, carmellose calcium, calcium carbonate, sodium hydrogen carbonate, sodium alginate, etc., lubricant such as magnesium stearate, talc, hydrogenated plant oil, Macrogol, silicone oil, etc., bonding agent such as polyvinyl alcohol, hydroxypropyl cellulose, methyl cellulose, ethyl cellulose, carmellose, gelatin, starch paste, etc., surfactant such as fatty acid ester, etc., plasticizer such as glycerol, and the like.

Preparation suitable for parenteral administration such as an injection preparation preferably comprises a sterilized aqueous preparation which is isotonic to blood of the person to be administered containing hydroxyproline or N-acyl derivative of hydroxyproline or a salt thereof. For example, in the case of an injection preparation, a solution for injection is prepared using a salt solution, a glucose solution or a carrier comprising a mixture of a salt solution and a glucose solution and the like.

In such a parenteral preparation, it is also possible to add one or more auxiliary component (s) selected from diluent, antiseptic agent, flavor, excipient, lubricant, bonding agent, surfactant, plasticizer, etc. which were exemplified for an oral preparation already.

The dose and the administering frequency of the preparation of the present invention vary depending upon dosage form and age, body weight, nature of symptom to be treated or degree of severeness of a patient and, usually, the preparation is administered once to several times a day so that the dose as hydroxyproline or N-acyl derivative of hydroxyproline or a salt thereof is made 5 mg to 5,000 mg or, preferably, 50 mg to 5,000 mg a day for an adult.

Although there is no particular limitation for the administering period, it is usually from 1 day to 1 year and, preferably, from 2 weeks to 3 months.

The preparation of the present invention is able to be used not only to human being but also to animals except human being (hereinafter, abbreviated as non-human animals).

As the non-human animals, mention may be made of mammals, birds, reptiles, amphibians, fish and animals other than human being.

The dose in the case of administration to non-human animals varies depending upon age and type of the animal and nature or degree of severeness of symptom and, usually, the preparation is administered once to several times a day so that the dose as hydroxyproline or N-acyl derivative of hydroxyproline or a salt thereof is made 0.5 mg to 500 mg or, preferably, 5 mg to 500 mg a day per kg of body weight.

Although there is no particular limitation for the administering period, it is usually from 1 day to 1 year and, preferably, from 2 weeks to 3 months.

By the same method as in the case of the preparation of the present invention, it is possible to prepare food and drink additives comprising hydroxyproline or N-acyl derivative of hydroxyproline or a salt thereof as an active ingredient.

If necessary, other food and drink additives is mixed with and dissolved in the food and drink additives of the present invention whereupon it is possible to make into the form of, for example, powder, granules, pellets, tablets and various liquid preparations.

The foods and drinks of the present invention are able to be processed and manufactured by the conventional method for the manufacture of foods and drinks except that hydroxyproline or N-acyl derivative of hydroxyproline or a salt thereof or a food and drink additive of the present invention is added to foods and drinks.

The foods and drinks of the present invention are also able to be produced by using granulating methods such as fluidized bed granulation, stirring granulation, extrusion granulation, rolling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method, and extrusion methods using an extruding granulator or an extruder.

The foods and drinks of the present invention may be in any of the forms including juice, refreshing soft drinks, tea, lactic acid beverages, dairy products such as fermented milk, frozen dessert, butter, cheese, yogurt, processed milk and skim milk, meat products such as ham, sausages and hamburger, fish products such as steamed, baked or fried fish paste, egg products such as baked or steamed foods made of beaten eggs, confectionery such as cookies, jellies, chewing gum, candies and snacks, bread, noodles, pickles, smoked foods, dried fish, preserved foods boiled down in soy sauce, salted foods, soups, seasonings, etc.

Furthermore, the foods and drinks of the present invention may take the form of a powdered food, a sheet-shaped food, a bottled food, a canned food, a retort food, a capsule food, a tablet food, a liquid food, a health drink, etc.

The foods and drinks of the present invention are able to be used as a health food and drink or a functional food and drink having an effect for anti-obesity.

To the foods and drinks or food and drink additives of the present invention may be added food additives which are commonly used in foods and drinks such as sweeteners, coloring agents, preservatives, thickening stabilizers, antioxidants, color developing agents, bleaching agents, fungicides, gum bases, bittering agents, enzymes, glazing agents, acidulants, seasonings, emulsifiers, nutrient supplements, additional materials for preparation, flavors, spice extracts, etc. which are mentioned, for example, in "Handbook for Indication of Food Additives" (Japan Food Additives Association, published on January 6, 1997).

Adding amount of hydroxyproline or N-acyl derivative of hydroxyproline or a salt thereof or of the food and drink additives to the foods and drinks of the present invention may be appropriately selected depending upon the type of foods and drinks, effect expected by ingestion of said foods and drinks, etc. and, usually, it is added so as to contain 0.1% by weight to 100% by weight or, preferably, 1.0% by weight to 100% by weight therein as hydroxyproline or N-acyl derivative of hydroxyproline or a salt thereof.

Depending upon ingestion form, age and body weight of a person to whom it is ingested, etc., the foods and drinks of the present invention is orally administered or, in other words, ingested once to several times a day so that amount as hydroxyproline or N-acyl derivative of hydroxyproline or a salt thereof is made 5 mg to 5,000 mg or, preferably, 50 mg to 5,000 mg a day to an adult.

Although there is no particular limitation for the ingesting period, it is usually from 1 day to 1 year, preferably, from 2 weeks to 3 months.

By the same method as in the case of the food and drink additives of the present invention, it is possible to prepare a feed additive comprising hydroxyproline or N-acyl derivative of hydroxyproline or a salt thereof as an active ingredient. If necessary, other feed additive is mixed with and dissolved in the feed additives of the present invention whereupon it is possible to make into the form of, for example, powder, granules, pellets, tablets and various liquid preparations.

The feed of the present invention is able to be processed and manufactured by the conventional method for the manufacture of feed except that hydroxyproline or N-acyl derivative of hydroxyproline or a salt thereof or a feed additive of the present invention is added to feed for non-human animals.

The feed for non-human animals include any feed for non-human feed for mammals, birds, reptiles, amphibians, fish, etc. and its examples include feed for pets such as dogs, cats, mice, etc., feed for livestock such as cows, pigs, etc., feed for poultry such as hens, turk, etc. and feed for cultivated fish such as sea breams, young yellowtails, etc. , and the like.

Examples of the feed to which hydroxyproline or N-acyl derivative of hydroxyproline or a salt thereof or the feed additive of the present invention is to be added include cereals, chaff and bran, vegetable oil cakes, animal-based feed materials, other feed materials, purified products thereof, etc.

As the cereals, mention may be made of milo, wheat, barley, oats, rye, brown rice, buckwheat, foxtail millet, broomcorn millet, Japanese millet, corn, soybean, etc.

As the chaff and bran, mention may be made of rice bran, defatted rice bran, wheat bran, wheat middlings, wheat germ, barley bran, pellet, corn bran, corn germ, etc.

As the vegetable oil cakes, mention may be made of soybean oil cake, soybean flour, linseed oil cake, cottonseed oil cake, peanut oil cake, safflower oil cake, coconut oil cake, palm oil cake, sesame oil cake, sunflower oil cake, rapeseed oil cake, kapok oil cake, mustard seed oil cake, etc.

As the animal-based feed materials, mention may be made of fish powder (such as northern ocean meal, imported meal, whole meal and coastal meal), fish soluble, meat powder, meat and bone powder, blood powder, degraded hair, bone powder, treated by-products for livestock, feather meal, silkworm pupa, skim milk, casein, dry whey, etc.

As other feed materials, mention may be made of stalks and leaves of plants (such as alfalfa, hay cube, alfalfa leaf meal, powder of false acacia, etc.), by-products from the corn processing industry (such as corn gluten meal, corn gluten feed, corn steep liquor, etc.), processed starch products (such as starch, etc.), sugar, products from the fermentation industry (such as yeast, beer cake, malt root, alcohol cake, soy sauce cake, etc.), agricultural by-products (such as processed citrus fruit cake, tofu cake, coffee cake, cocoa cake, etc.), cassava, broad bean, guar meal, seaweeds, krill, spirulina, chlorella, minerals, etc.

As the purified products thereof, mention may be made of proteins (such as casein, albumin, etc.), amino acids, saccharides (such as starch, cellulose, sucrose, glucose, etc.), minerals, vitamins, etc.

The feed of the present invention is also to be produced by using granulating methods such as fluidized bed granulation, stirring granulation, extrusion granulation, tumbling granulation, air stream granulation, compression molding granulation, disruption granulation, spray granulation and blasting granulation, coating methods such as pan coating, fluidized bed coating and dry coating, plumping methods such as puff drying, excess steam method, foam mat method and microwave heating method and extrusion methods using an extruding granulator or an extruder.

The feed of the present invention is able to be used as a feed for anti-obesity.

Adding amount of hydroxyproline or N-acyl derivative of hydroxyproline or a salt thereof or of the feed additive to the feed of the present invention may be appropriately selected depending upon the type of feed, effect expected by ingestion of said feed, etc. and, usually, it is added so as to contain 0.1% by weight to 100% by weight or, preferably, 1.0% by weight to 100% by weight therein as hydroxyproline or N-acyl derivative of hydroxyproline or a salt thereof.

When the feed of the present invention is ingested to non-human animals, depending upon ingestion form, type of the ingesting animals, age and body weight of the animal, etc., the feed is orally administered or, in other words, ingested once to several times a day so that amount as hydroxyproline or N-acyl derivative of hydroxyproline or a salt thereof is made 0.5 mg to 500 mg or, preferably, 5 mg to 500 mg a day to an adult.

Although there is no particular limitation for the ingesting period, it is usually from 1 day to 1 year and, preferably, from 2 weeks to 3 months.

When hydroxyproline or N-acyl derivative of hydroxyproline or a salt thereof is administered to human being or non-human animals by the above-mentioned method, it is possible to suppress obesity.

### Best Mode for Carrying Out the Invention

### Example 1

KK-Ay/Ta Jcl mice (Clea Japan, Inc.; male; six weeks age) (15 mice) of an obesity type 2 diabetic model were divided into three groups each comprising five and named group 1 to group 3.

The mice of the groups 1 to 3 were made free to take feed and water. A commercially available feed CE-2 (manufactured by Clea Japan, Inc.) was ingested to the mice of group 1. CE-2 to which 1% by weight of trans-4-hydroxy-L-proline (manufactured by Kyowa Hakko Kogyo; hereinafter, abbreviated as the hydroxyproline) was added was ingested to the mice of group 2. CE-2 to which 1% by weight of trans-N-acetyl-4-hydroxy-L-proline (manufactured by Kyowa Hakko Kogyo; hereinafter, abbreviated as the N-acetylhydroxyproline) was added was ingested to the mice of group 3.

Body weight on the initial day of the test and on the 17th day and amount of the ingested feed from the initial day of the test to the 17th day were measured. As an index for the progress of obesity, a body weight increase during the test period for the total ingested feed amount during the test period was calculated for each mouse. The value was shown in terms of mean value ± standard error (n = 5) and statistic ratio of risk (p value) was determined by a t-test.

Results of the above calculation are shown in Table 1.

**Table 1**

| | Body Weight Increase/Ingested Feed Amount (%) |
|---|---|
| Group 1 | 8.1 ± 0.6 |
| Group 2 | 5.8 ± 0.4 |
| Group 3 | 4.2 ± 0.6* |

| | |
|---|---|
| (*: p < 0.05, to the group 1) | |

It is apparent from Table 1 that, as compared with the body weight increase per ingested feed amount of the group 1, the body weight increase per ingested feed amount of the group 3 was a significantly low. The body weight increase per ingested feed amount of the group 2 also tended to show low value as compared with the body weight increase per ingested feed amount of the group 1. From the result as such, an anti-obesity action of the hydroxyproline and the N-acetylhydroxyproline is apparent.

### Example 2

Water was added to a composition having the following formulation mentioned in Table 2 to make 1,000 ml whereupon a refreshing soft drink (for ten bottles) for anti-obesity was prepared.

**Table 2**

| Composition | Amount |
|---|---|
| Hydroxyproline | 5 g |
| Vitamin C | 1 g |
| Vitamin B₁ | 5 mg |
| Vitamin B₂ | 10 mg |
| Vitamin B₆ | 25 mg |
| Liquid Sugar | 150 g |
| Citric Acid | 3 g |
| Flavor | 1 g |

### Example 3

A composition having the formulation mentioned in Table 3 was extracted with 1,000 ml of water to prepare 1,000 ml of tea beverage for anti-obesity.

**Table 3**

| Composition | Amount |
|---|---|
| Hydroxyproline | 5 g |
| Tea Leaves | 15 g |

### Example 4

According to the formulation mentioned in Table 4, chewing gum (for 30 pieces) for anti-obesity was prepared.

**Table 4**

| Composition | Amount |
|---|---|
| Hydroxyproline | 1.5 g |
| Gum Base | 25 g |
| Sugar | 63 g |
| Starch Syrup | 10 g |
| Flavor | 1 g |

### Example 5

According to the formulation mentioned in Table 5, candy (for 20 products) for anti-obesity was prepared.

**Table 5**

| Composition | Amount |
|---|---|
| Hydroxyproline | 1 g |
| Sugar | 80 g |
| Starch Syrup | 20 g |
| Flavor | 0.1 g |

### Example 6

According to the formulation mentioned in Table 6, tablets (200 mg per tablet) for anti-obesity were prepared by a conventional method.

**Table 6**

| Composition | Amount |
|---|---|
| Hydroxyproline | 50 mg |
| Lactose | 90 mg |
| Corn Starch | 30 mg |
| Synthetic Aluminum Silicate | 12 mg |
| Carboxymethylcellulose Calcium | 15 mg |
| Magnesium Stearate | 3 mg |

### Example 7

According to the formulation mentioned in Table 7, a powder (550 mg per chartula) for anti-obesity was prepared.

**Table 7**

| Composition | Amount |
|---|---|
| N-Acetylhydroxyproline | 50 mg |
| Lactose | 300 mg |
| Corn Starch | 200 mg |

### Example 8

According to the formulation mentioned in Table 8, a hard capsule preparation (160 mg per capsule) for anti-obesity was prepared.

**Table 8**

| Composition | Amount |
|---|---|
| Hydroxyproline | 50 mg |
| Lactose | 60 mg |
| Corn Starch | 30 mg |
| Hydroxypropyl Cellulose | 20 mg |

To 50 mg of hydroxyproline were added 60 mg of lactose and 30 mg of corn starch, and mixing was carried out. An aqueous solution of 20 mg of hydroxypropyl cellulose was added thereto and the mixture was kneaded. Then, granules were prepared using an extruding granulator. The granules were filled in gelatin hard capsules to prepare a hard capsule preparation.

### Example 9

According to the formulation mentioned in Table 9, a soft capsule preparation (170 mg per capsule) for anti-obesity was prepared.

**Table 9**

| Composition | Amount |
|---|---|
| N-Acetylhydroxyproline | 50 mg |
| Soybean Oil | 120 mg |

To 120 mg of soybean oil was added 50 mg of N-Acetylhydroxyproline, and mixing was carried out. Then, the mixture was filled in soft capsules using an automated molding machine of a rotary dies type by a conventional method whereupon a soft capsule preparation was prepared.

### Industrial Applicability

In accordance with the present invention, there is provided an anti-obesity agent comprising hydroxyproline, N-acyl derivative of hydroxyproline or a pharmaceutically acceptable salt thereof as an active ingredient or a food and drink, a food and drink additive, a feed or a feed additive for anti-obesity comprising the same.

## Claims

1. An anti-obesity agent, which comprises as an active ingredient, hydroxyproline or N-acyl derivative of hydroxyproline or a pharmaceutically acceptable salt thereof.

2. A food and drink or a food and drink additive for anti-obesity, which comprises as an effective ingredient, hydroxyproline or N-acyl derivative of hydroxyproline or a pharmaceutically acceptable salt thereof.

3. A feed or a feed additive for anti-obesity, which comprises as an active ingredient, hydroxyproline or N-acyl derivative of hydroxyproline or a pharmaceutically acceptable salt thereof.

4. A method for suppressing obesity, which comprises administering hydroxyproline or N-acyl derivative of hydroxyproline or a pharmaceutically acceptable salt thereof.

5. Use of hydroxyproline or N-acyl derivative of hydroxyproline or a pharmaceutically acceptable salt thereof for the manufacture of an anti-obesity agent.

6. Use of hydroxyproline or N-acyl derivative of hydroxyproline or a pharmaceutically acceptable salt thereof for the manufacture of foods and drinks or food and drink additives for anti-obesity.

7. Use of hydroxyproline or N-acyl derivative of hydroxyproline or a pharmaceutically acceptable salt thereof for the manufacture of feeds or feed additives for anti-obesity.
